# EUROPÄISCHE PATENTSCHRIFT

(11) **EP 2 529 763 B1**
(45) Veröffentlichungstag und Bekanntmachung des Hinweises auf die Patenterteilung: **12.10.2016**
(21) Anmeldenummer: 11168635.8
(22) Anmeldetag: 03.06.2011
(51) Int. Cl.: A61L 27/30, A61L 27/32, A61L 27/54

(54) **Verfahren zum Herstellen einer Implantatbeschichtung und entsprechendes Implantat**
Method for manufacturing an implant coating and implant
Procédé de fabrication d'un revêtement d'implant et implant associé

(43) Veröffentlichungstag der Anmeldung: 05.12.2012
(73) Patentinhaber: Waldemar Link GmbH & Co. KG, 22339 Hamburg (DE)
(72) Erfinder: LINK, Helmut D., 22397 Hamburg (DE)
(74) Vertreter: Glawe, Delfs, Moll

(56) Entgegenhaltungen:
- EP-A2- 0 395 187
- US-A1- 2004 131 754

## Beschreibung

Die Erfindung betrifft ein Verfahren zum Herstellen einer Implantatbeschichtung und ein entsprechendes Implantat. Die Implantate sollen in der Human- und Tiermedizin zur Behandlung von Knochendefekten verwendet werden und umfassen sowohl temporäre als auch dauerhafte Implantate. Bei dem Implantat kann es sich beispielsweise um eine im Körper verbleibende Prothese, eine sogenannte Endoprothese, handeln.

Jedes medizinische Implantat stellt für den Implantatträger einen Fremdkörper dar und bewirkt daher eine komplexe biologische Interaktion auf verschiedensten Ebenen. Eine der wichtigsten Reaktionen des Körpers ist Rekrutierung osteogener Stammzellen an der Implantatoberfläche, die sogenannte Osteokonduktion. Dabei absorbiert die Implantatoberfläche in einem ersten Schritt Fibrinogen, an dem sich Blutplättchen anheften, die ihrerseits aktiviert osteogene Wachstumsfaktoren freisetzen und zu einer Wanderung osteogener Stammzellen zum Implantat, genauer zu der Implantatoberfläche führen. Die osteogenen Stammzellen scheiden eine organische Knochenmatrix ab, die durch Calciumphosphatablagerung mineralisiert wird. Im Idealfall ist das Implantat nach abgeschlossener Primärstabilität verleihender Osteokonduktion und Sekundärstabilität verleihender Osteointegration in festem Verbund mit dem Knochen.

Es ist bekannt, dass die Rauhigkeit der Implantatoberfläche den Prozess der Osteokonduktion beeinflusst, wobei mit zunehmender Rauhigkeit, beispielsweise durch Beschichtung des Implantats mittels einer dünnen Calciumphosphatschicht, mit einer besseren Osteointegration einhergeht. Erste experimentelle Ergebnisse an Tieren zeigen eine verbesserte Osteointegration der mit Calciumphosphat beschichteten Implantate im Vergleich zu unbeschichteten Kontrollimplantaten (Junker et al., Effects of implant surface coatings and composition on bone integration: a systematic review. Clinical Oral Implants Research, Volume 20 Issue Supplement 4: 185-206, September 2009). Offensichtlich wirkt das Calciumphosphat sowohl osteokonduktiv als auch osteoinduktiv, d.h. es dient einerseits als Stützstruktur für die Osteoblasten und fördert andererseits die Knochenneubildung, d.h. das Anwachsen des Implantats am Knochen. Darüber hinaus maskiert der Calciumphosphatüberzug das künstliche Implantat, so dass es nicht mehr als Fremdkörper erkannt wird.

Aus der klinischen Praxis ist ebenfalls bekannt, dass jegliches Implantat ein bevorzugter Untergrund für die Ansiedlung von Bakterien ist. Bestimmte Bakterien wie *Staphylococcus aureus* sind in der Lage, auf dem Implantat einen Biofilm aus extrazellulärem Schleim zu bilden, in dem sich bakterielle Mikrokolonien bilden und sich vermehren, bis der Biofilm das gesamte Implantat überzogen hat. Mit zunehmender bakterieller Infektion, die sich über Jahre hinziehen kann und häufig mit einer Lockerung des Implantats einhergeht, ist eine systemische Behandlung mit Antibiotika in den seltensten Fällen erfolgreich, da der Biofilm für die Bakterienkolonien einen "Schutzwall" bildet, hinter dem sich selbst bei hoch dosierter systemischer Anwendung von Antibiotika keine therapeutisch wirksamen Antibiotika-Konzentrationen einstellen. Auch erreichen systemisch verabreichte Antibiotika die Umgebung des Implantats kaum, da das Gewebe am Implantat häufig vernarbt und damit schlecht durchblutet ist. Im Ergebnis muss eine chirurgische Sanierung stattfinden, d.h. das Implantat muss entfernt und die bakterielle Infektion vor Ort behandelt werden.

Um hohe Antibiotika-Wirkspiegel am Implantat sicherzustellen, die einer Besiedelung durch Bakterien und insbesondere einer Biofilmbildung vorbeugen und um einer gegebenenfalls nachfolgenden bakteriellen Infektion entgegenzuwirken, ist es von Nutzen, das Implantat mit Antibiotika zu beschichten. Aus dem Stand der Technik ist bekannt, Antibiotika mittels Bindemittel oder eingebettet in eine organische Matrix auf der porösen Oberfläche des Implantats als Schicht aufzubringen, wobei das Implantat vollflächig überschichtet ist (Moskowitz et al., The effectiveness of the controlled release of gentamicin from polyelectrolyte multilayer in the treatment of Staphylococcus aureus infection in rabbit bone model, Biomaterials (2010) volume 31, issue 23: 6019-6030, August 2010; Vester et al., Gentamycin delivered from a PDLLA coating of metallic implants In vivo and in vitro characterisation for local prophylaxis of implantrelated osteomyelitis, Injury 2010: 1053-1059; DE 10 2005 002 703).

Der Erfindung liegt die Aufgabe zugrunde, medizinische Implantate zu schaffen, die möglichst infektionsfrei und unter Bildung eines festen Verbundes mit dem Knochen einheilen.

Insbesondere besteht die Aufgabe der Erfindung darin, eine antibiotische Implantatbeschichtung zu entwickeln, die einerseits wirksame Mengen an Antibiotika lokal an der Grenzfläche zwischen Implantat und dem Gewebe aus einer entsprechenden Antibiotika-Beschichtung freisetzt (Ziel 1), andererseits aber auch nicht den Zugang zu der unter der Antibiotika-Beschichtung liegenden osteoinduktiv und osteokonduktiv wirkenden Deckschicht auf der Basis von Calciumphosphat und/oder Calciumcarbonat verwehrt (Ziel 2).

Dieser auf den ersten Blick unauflösbare Zielkonflikt wird überraschenderweise mit der erfindungsgemäßen Lösung der Merkmale der unabhängigen Ansprüche gelöst. In den jeweiligen Unteransprüchen sind vorteilhafte und zweckmäßige Weiterbildungen angegeben.

Es ist ein Verfahren zur Herstellung einer Beschichtung auf einem medizinischen Implantat und ein entsprechendes Implantat vorgesehen, umfassend die Beschichtung von mindestens einem Teil der Oberfläche des medizinischen Implantats mit einer osteoinduktiven und/oder osteokonduktiven Deckschicht auf der Basis von Calciumphosphat und/oder Calciumcarbonat, wobei erfindungsgemäß vorgesehen ist, dass die osteoinduktive und/oder osteokonduktive Schicht mit einem in wässerigem Milieu löslichen antibiotischen Wirkstoff derart überschichtet wird, dass unter Freilassung von Zwischenräumen auf der osteoinduktiven und/oder osteokonduktiven Deckschicht Flecken gebildet werden.

Mit dem erfindungsgemäßen Verfahren und dem nach diesem Verfahren hergestellten Implantat wird es ermöglicht, den oben genannten Zielkonflikt zu vermeiden und in vollem Umfang die Vorteile einer Antibiotikaschicht und einer osteoinduktiven und/oder osteokonduktiven Deckschicht auf der Basis von Calciumphosphat und/oder Calciumcarbonat miteinander zu kombinieren, was im Ergebnis zu einer infektionsfreien Einheilung des Implantats unter Ausbildung eines festen Implantat-Knochen-Verbundes führt.

Die fleckenförmige Überschichtung der osteoinduktiven und/oder osteokonduktiven Deckschicht im Rahmen der vorliegenden Erfindung bedeutet, dass die Deckschicht mit Stellen versehen ist, auf die der antibiotische Wirkstoff bzw. die den Wirkstoff tragende Schicht oder Matrix aufgetragen ist. Dementsprechend weisen die Freilassungen zwischen den Flecken keinen antibiotischen Wirkstoff auf. Im Sinne der Erfindung besteht die fleckenförmige Überschichtung aus einem Kollektiv einzelner Flecken bzw. Freilassungen unterschiedlicher Abmessungen und Formen. Vorzugsweise decken die Flecken mit dem antibiotischen Wirkstoff zwischen 1% und 95%, vorzugsweise zwischen 5% und 90%, weiter vorzugsweise zwischen 10% und 85%, weiter vorzugsweise zwischen 15% und 80%, weiter vorzugsweise zwischen 20% und 75%, weiter vorzugsweise zwischen 25% und 75%, weiter vorzugsweise zwischen 30% und 70%, weiter vorzugsweise zwischen 35% und 65%, weiter vorzugsweise zwischen 40% und 60%, weiter vorzugsweise zwischen 45% und 55%, weiter vorzugsweise etwa 50% der darunter liegenden osteoinduktiven und/oder osteokonduktiven Deckschicht ab. Die Größe der Flecken kann variieren, wobei man bei einer tropfenförmigen Beaufschlagung der osteoinduktiven und/oder osteokonduktiven Deckschicht vorzugsweise Flecken mit einem Durchmesser von 0,5-20 mm, 0,5-15 mm, 0,5-10 mm, 0,5-5 mm, 0,5-4 mm, 0,5-3 mm, 0,5-2 mm, 0,5-1mm, 1-20 mm, 1-15 mm, 1-10 mm, 1-5 mm, 1-4 mm, 1-3 mm, 1-2 mm erhält. Der mittlere Fleckendurchmesser kann in einem Bereich von 0,75-20 mm, 1-20 mm, 2-15 mm, 3-10 mm und 4-5 mm liegen. Die vorgenannten Parameter der Flächenabdeckung als auch Fleckengrößen können miteinander kombiniert werden.

Gemäß einer bevorzugten Ausführungsform der Erfindung kann die osteoinduktive und/oder osteokonduktive Deckschicht auf der Basis von Calciumphosphat Hydroxylapatit umfassen. Hydroxylapatit ist ein resorbierbares Biomaterial, das sich als Werkstoff für Knochenersatz in der Praxis bereits vielfach bewährt hat und in diesem Zusammenhang überwiegend als Beschichtungsmaterial eingesetzt wird, um von den Vorteilen seiner osteinduktiven und osteokonduktiven Wirkung Gebrauch zu machen. Es können jedoch auch andere Calciumphosphatschichten, beispielsweise α- und/oder β-Tricalciumphosphat, Tetracalciumphosphat oder Mischungen dieser Varianten, gegebenenfalls mit Zusätzen von Calciumoxid, verwendet werden.

Grundsätzlich kommt im Rahmen der vorliegenden Erfindung jeder antibiotische Wirkstoff in Betracht, der unter *in-vivo* Anwendungsbedingungen, d.h. insbesondere bei Körpertemperatur und in wässerigem Milieu, seine antibakterielle Wirkung entfaltet. In der medizinischen Praxis haben sich insbesondere Aminoglycosid-Antibiotika, vorzugsweise Gentamicin und Amikacin, aber auch Apramycin, Geneticin (G418), Kanamycin, Netilmicin, Neomycin, Paromomycin, Spectinomycin, Streptomycin, Tobramycin; Lincosamid-Antibiotika, vorzugsweise Clindamycin, Lincomycin; Cephalosporine-Antibiotika, vorzugsweise Cefuroxim und Cefoperazon; Fluorchinolone-Antibiotika, vorzugsweise Ofloxazin; Glycopeptid-Antibiotika, vorzugsweise Vancomycin; β-Lactam-Antibiotika, vorzugsweise Ampicillin und deren entsprechende Salze bei der Bekämpfung bakterieller Infektionen durchgesetzt.

Von besonderer praktischer Relevanz ist das Aminoglycosid-Antibiotika Gentamicin, das dem für Infektionen besonders bedeutsamen *Staphylococcus aureus* Stämmen entgegenwirkt, welche insbesondere auch wesentlich an der Ausbildung des Biofilm-"Schutzwalls" beteiligt sind, wie eingangs bereits dargestellt wurde. Da jedoch auch das antibiotische Wirkspektrum von Gentamicin nicht lückenlos ist und insbesondere die Gefahr von erworbener Gentamicin-Resistenz besteht, ist eine Ergänzung von Gentamicin durch weitere Antibiotika von Vorteil. Resistenzen gegen häufig verwendete Antibiotika sind insbesondere im Krankenhausbereich regelmäßig anzutreffen und häufig nur durch Kombination mehrerer Antibiotika mit verschiedenen Wirkmechanismen mit Erfolg zu bekämpfen. So kann beispielsweise Gentamicin mit dem Lincosamid-Antibiotika Clindamycin kombiniert werden um synergistisch gegen Staphylokokken, Streptokokken und Propionbakterien zu wirken. Ein ähnliches Wirksamkeitsspektrum weist die Kombination aus Gentamicin und dem Cephalosporine-Antibiotika Cefuroxim auf. Um *Pseudomans* Infektionen vorzubeugen, kann auf eine Kombination aus Gentamicin, Fluorchinolone-Antibiotika Ofloxazin oder Cefoperazon und weiteren Aminoglycosid-Antibiotika Amikacin zurückgegriffen werden. In der klinischen Praxis von besonderer Relevanz sind ebenfalls die zwischenzeitlich vermehrt in Krankenhäusern auftretenden Methicillin-resistenten *Staphylococcus aureus* (MRSA) und Methicillin-resistenten *Staphylococcus epidermidis* (MRSE) Stämme, die mit einer Kombination von Gentamicin, dem Glycopeptid-Antibiotika Vancomycin und dem Fluorchinolone-Antibiotika Ofloxazin mit Aussicht auf Erfolg bekämpft werden können. Bei einer *Enterococcus* Infektion als auch im Kampf gegen Vancomycin-Resistenzen ist eine Kombination aus Vancomycin, Gentamicin und dem β-Lactam-Antibiotika Ampicillin hilfreich. Unabhängig von der Verwendung von Antibiotika auf dem erfindungsgemäßen Implantat kann es ebenfalls von Vorteil sein, präventiv und/oder nach der Implantierung des Implantats eine begleitende systemische Antibiotika-Therapie durchzuführen.

Gemäß einer bevorzugten Ausführungsform der Erfindung haften die Antibiotika oder deren Salze selbst oder über einen Träger, vorzugsweise einen (z.B. polymeren) Schichtbildner, oder eingebettet in eine Matrix auf der osteoinduktiven und/oder osteokonduktiven Schicht. Der Träger oder die Matrixbildner können beispielsweise aus Stearinsäure, Palmitinsäure, Myristinsäure, Behensäure, Myristylpalmitat, Cetylpalmitat oder Cerylcerotinat aufgebaut sein, die gut auf Metall- und Kunststoffoberflächen haften.

Im Rahmen der Erfindung kommen jegliche Antibiotika-Salze in Betracht, einschließlich in Wasser lösliche Salze des Gentamicins, des Sisomycins, des Netilmicins, des Streptomycins, des Tobramycins, des Spectinomycins, des Vancomycins, des Ciprofloxacins, des Moxifloxacins, des Clindamycins, des Lincomycins, des Tetracyclins, des Chlortetracyclins, des Oxytetracyclins und des Rolitetracyclins, wobei Gentamicin-Salze der Palmitinsäure, der Laurinsäure, der Stearinsäure, der Ölsäure, der Phenylbuttersäure, der Naphtalen-1-carbonsäure oder Sulfate des Gentamicins bevorzugt sind.

Es kann von Vorteil sein, die gewünschte retardierende Wirkstofffreisetzung über eine geringe oder schwere Löslichkeit des antibiotischen Wirkstoffs in wässerigem Milieu sicher zu stellen, beispielsweise durch Verwendung von in wässerigem Milieu schwer oder gering löslichen Antibiotika-Salzen. Beispielhaft genannt seien die Antibiotika-Salze aus der Gruppe des Netilmicin-Laurates, des Netilmicin-Dodecylsulfates, des Netilmicin-Myristates, des Sisomicin-Laurates, des Sisomicin-Myristates, der Sisomicin-Dodecylsulfates, des Gentamicin-Laurates, des Gentamicin-Myristates, des Clindamycin-Laurates, des Amikacin-Laurates, des Amikacin-Myristates, des Amikacin-Dodecylsulfates, des Kanamycin-Laurates, des Kanamycin-Myristates, des Kanamycin-Dodecylsulfates, des Vancomycin-Laurates, des Vancomycin-Dodecylsulfates, des Vancomycin-Myristates, des Vancomycin-Teicoplanins, des Tobramycin-Laurates, des Tobramycin-Myristates, des Tobramycin-Dodecylsulfates, des Ciprofloxacin-Laurates, des Ciprofloxacin-Myristates, des Clindamycin-Teicoplanins, des Fusidinsäure-Gentamicins, des Fusidinsäure-Sisomycins, des Fusidinsäure-Netilmicin, des Fusidinsäure-Streptomycins, des Fusidinsäure-Tobramycins, des Fusidinsäure-Spectinomycins, des Fusidinsäure-Vancomycins, des Fusidinsäure-Ciprofloxacins, des Fusidinsäure-Moxifloxacins, des Fusidinsäure-Clindamycins, des Fusidinsäure-Lincomycins, des Fusidinsäure-Tetracyclins, des Fusidinsäure-Chlortetracyclins, des Fusidinsäure-Oxytetracyclins und des Fusidinsäure-Rolitetracyclins. Die gering löslichen Salze können in geeigneten organischen Lösungsmitteln gelöst und mit diesen Lösungen, gegebenenfalls unter Hinzufügung eines Schichtbildners oder einer einbettenden Matrix auf die osteoinduktive und/oder osteokonduktive Deckschicht aufgetragen werden.

## Patentansprüche

1. Medizinisches Implantat, das zumindest auf einem Teil seiner Oberfläche eine Beschichtung mit einer osteoinduktiven und/oder osteokonduktiven Deckschicht auf der Basis von Calciumphosphat und/oder Calciumcarbonat aufweist,
**dadurch gekennzeichnet, dass**
die osteoinduktive und/oder osteokonduktive Deckschicht fleckenförmig unter Freilassung von Zwischenräumen auf der osteoinduktiven und/oder osteokonduktiven Deckschicht mit einem in wässerigem Milieu schwer oder gering löslichen antibiotischen Wirkstoff überschichtet ist.

2. Medizinisches Implantat nach Anspruch 1,
**dadurch gekennzeichnet, dass**
die osteoinduktive und/oder osteokonduktive Deckschicht auf der Basis von Calciumphosphat Hydroxylapatit umfasst.

3. Medizinisches Implantat nach einem der vorangehenden Ansprüche,
**dadurch gekennzeichnet, dass**
der antibiotische Wirkstoff mindestens ein Antibiotika, ausgewählt aus der Gruppe bestehend aus:
- Aminoglycosid-Antibiotika, vorzugsweise Gentamicin und Amikacin;
- Lincosamid-Antibiotika, vorzugsweise Clindamycin, Lincomycin;
- Cephalosporine-Antibiotika, vorzugsweise Cefuroxim und Cefoperazon;
- Fluorchinolone-Antibiotika, vorzugsweise Ofloxazin,
- Glycopeptid-Antibiotika, vorzugsweise Vancomycin,
- β-Lactam-Antibiotika, vorzugsweise Ampicillin,
oder deren Salze umfasst.

4. Medizinisches Implantat nach Anspruch 3,
**dadurch gekennzeichnet, dass**
das Antibiotika oder deren Salze unmittelbar oder über einen Träger, vorzugsweise einen polymeren Schichtbildner, auf der osteoinduktiven und/oder osteokonduktiven Schicht haften.

5. Medizinisches Implantat nach einem der Ansprüche 3 und 4,
**dadurch gekennzeichnet dass**
die Antibiotika-Salze Gentamicin-Salze der Palmitinsäure, der Laurinsäure, der Stearinsäure, der Ölsäure, der Phenylbuttersäure, der Naphtalen-1-carbonsäure oder Sulfate des Gentamicins sind.

6. Medizinisches Implantat nach einem der vorangehenden Ansprüche,
**dadurch gekennzeichnet, dass**
der in wässerigem Milieu schwer oder gering lösliche antibiotische Wirkstoff ein Antibiotika-Salz ausgewählt aus der Gruppe des Netilmicin-Laurates, des Netilmicin-Dodecylsulfates, des Netilmicin-Myristates, des Sisomicin-Laurates, des Sisomicin-Myristates, der Sisomicin-Dodecylsulfates, des Gentamicin-Laurates, des Gentamicin-Myristates, des Clindamycin-Laurates, des Amikacin-Laurates, des Amikacin-Myristates, des Amikacin-Dodecylsulfates, des Kanamycin-Laurates, des Kanamycin-Myristates, des Kanamycin-Dodecylsulfates, des Vancomycin-Laurates, des Vancomycin-Dodecylsulfates, des Vancomycin-Myristates, des Vancomycin-Teicoplanins, des Tobramycin-Laurates, des Tobramycin-Myristates, des Tobramycin-Dodecylsulfates, des Ciprofloxacin-Laurates, des Ciprofloxacin-Myristates, des Clindamycin-Teicoplanins, des Fusidinsäure-Gentamicins, des Fusidinsäure-Sisomycins, des Fusidinsäure-Netilmicin, des Fusidinsäure-Streptomycins, des Fusidinsäure-Tobramycins, des Fusidinsäure-Spectinomycins, des Fusidinsäure-Vancomycins, des Fusidinsäure-Ciprofloxacins, des Fusidinsäure-Moxifloxacins, des Fusidinsäure-Clindamycins, des Fusidinsäure-Lincomycins, des Fusidinsäure-Tetracyclins, des Fusidinsäure-Chlortetracyclins, des Fusidinsäure-Oxytetracyclins und des Fusidinsäure-Rolitetracyclins ist.

7. Medizinisches Implantat nach einem der vorangehenden Ansprüche,
**dadurch gekennzeichnet, dass**
die fleckenförmige Überschichtung der osteoinduktiven und/oder osteokonduktiven Schicht mit dem in wässerigem Milieu schwer oder gering löslichen antibiotischen Wirkstoff durch lokales Auftragen unter Freilassung von Zwischenräumen auf der osteoinduktiven und/oder osteokonduktiven Schicht erfolgt, vorzugsweise mittels Aufsprühen, Auftropfen oder Aufpipettieren einer den antibiotischen Wirkstoff enthaltenden Lösung oder Suspension.

8. Verfahren zur Herstellung einer Beschichtung auf einem medizinischen Implantat, umfassend die Schritte:
- Beschichtung von mindestens einem Teil der Oberfläche des medizinischen Implantats mit einer osteoinduktiven und/oder osteokonduktiven Deckschicht auf der Basis von Calciumphosphat und/oder Calciumcarbonat,
- Überschichtung der osteoinduktiven und/oder osteokonduktiven Schicht mit einem in wässerigem Milieu schwer oder gering löslichen antibiotischen Wirkstoff derart, dass unter Freilassung von Zwischenräumen auf der osteoinduktiven und/oder osteokonduktiven Deckschicht Flecken gebildet werden.

9. Verfahren nach Anspruch 8,
**dadurch gekennzeichnet, dass**
die osteoinduktive und/oder osteokonduktive Deckschicht auf der Basis von Calciumphosphat Hydroxylapatit umfasst.

10. Verfahren nach einem der Ansprüche 8 und 9,
**dadurch gekennzeichnet, dass**
der antibiotische Wirkstoff mindestens ein Antibiotika, ausgewählt aus der Gruppe bestehend aus:
- Aminoglycosid-Antibiotika, vorzugsweise Gentamicin und Amikacin;
- Lincosamid-Antibiotika, vorzugsweise Clindamycin, Lincomycin;
- Cephalosporine-Antibiotika, vorzugsweise Cefuroxim und Cefoperazon;
- Fluorchinolone-Antibiotika, vorzugsweise Ofloxazin,
- Glycopeptid-Antibiotika, vorzugsweise Vancomycin,
- β-Lactam-Antibiotika, vorzugsweise Ampicillin, oder deren Salze umfasst.

11. Verfahren nach Anspruch 10,
**dadurch gekennzeichnet, dass**
das Antibiotika oder deren Salze unmittelbar oder über einen Träger, vorzugsweise einen polymeren Schichtbildner, auf der osteoinduktiven und/oder osteokonduktiven Schicht haften.

12. Verfahren nach einem der Ansprüche 10 und 11,
**dadurch gekennzeichnet dass**
die Antibiotika-Salze Gentamicin-Salze der Palmitinsäure, der Laurinsäure, der Stearinsäure, der Ölsäure, der Phenylbuttersäure, der Naphtalen-1-carbonsäure oder Sulfate des Gentamicins sind.

13. Verfahren nach einem der Ansprüche 8-12,
**dadurch gekennzeichnet, dass**
der in wässerigem Milieu schwer oder gering lösliche antibiotische Wirkstoff ein Antibiotika-Salz ausgewählt aus der Gruppe des Netilmicin-Laurates, des Netilmicin-Dodecylsulfates, des Netilmicin-Myristates, des Sisomicin-Laurates, des Sisomicin-Myristates, der Sisomicin-Dodecylsulfates, des Gentamicin-Laurates, des Gentamicin-Myristates, des Clindamycin-Laurates, des Amikacin-Laurates, des Amikacin-Myristates, des Amikacin-Dodecylsulfates, des Kanamycin-Laurates, des Kanamycin-Myristates, des Kanamycin-Dodecylsulfates, des Vancomycin-Laurates, des Vancomycin-Dodecylsulfates, des Vancomycin-Myristates, des Vancomycin-Teicoplanins, des Tobramycin-Laurates, des Tobramycin-Myristates, des Tobramycin-Dodecylsulfates, des Ciprofloxacin-Laurates, des Ciprofloxacin-Myristates, des Clindamycin-Teicoplanins, des Fusidinsäure-Gentamicins, des Fusidinsäure-Sisomycins, des Fusidinsäure-Netilmicin, des Fusidinsäure-Streptomycins, des Fusidinsäure-Tobramycins, des Fusidinsäure-Spectinomycins, des Fusidinsäure-Vancomycins, des Fusidinsäure-Ciprofloxacins, des Fusidinsäure-Moxifloxacins, des Fusidinsäure-Clindamycins, des Fusidinsäure-Lincomycins, des Fusidinsäure-Tetracyclins, des Fusidinsäure-Chlortetracyclins, des Fusidinsäure-Oxytetracyclins und des Fusidinsäure-Rolitetracyclins ist.

14. Verfahren nach einem der Ansprüche 8-13,
**dadurch gekennzeichnet, dass**
die fleckenförmige Überschichtung der osteoinduktiven und/oder osteokonduktiven Schicht mit dem in wässerigem Milieu schwer oder gering löslichen antibiotischen Wirkstoff durch lokales Auftragen unter Freilassung von Zwischenräumen auf der osteoinduktiven und/oder osteokonduktiven Schicht erfolgt, vorzugsweise mittels Aufsprühen, Auftropfen oder Aufpipettieren einer den antibiotischen Wirkstoff enthaltenden Lösung oder Suspension.

15. Medizinisches Implantat erhältlich nach einem Verfahren der Ansprüche 8-14.

## Claims

1. Medical implant having on at least part of its surface a coating having an osteoinductive and/or osteoconductive cover layer based on calcium phosphate and/or calcium carbonate,
**characterized in that**
an active antibiotic ingredient which is slightly or poorly soluble in an aqueous environment overcoats the osteoinductive and/or osteoconductive cover layer in a patchy manner with spaces being left free on the osteoinductive and/or osteoconductive cover layer.

2. Medical implant according to Claim 1,
**characterized in that**
the osteoinductive and/or osteoconductive cover layer based on calcium phosphate comprises hydroxylapatite.

3. Medical implant according to either of the preceding claims,
**characterized in that**
the active antibiotic ingredient comprises at least one antibiotic selected from the group consisting of:
- aminoglycoside antibiotics, preferably gentamicin and amikacin;
- lincosamide antibiotics, preferably clindamycin, lincomycin;
- cephalosporin antibiotics, preferably cefuroxime and cefoperazone;
- fluoroquinolone antibiotics, preferably ofloxacin,
- glycopeptide antibiotics, preferably vancomycin,
- □-lactam antibiotics, preferably ampicillin, or the salts thereof.

4. Medical implant according to Claim 3,
**characterized in that**
the antibiotic or the salts thereof adhere(s) directly or via a support, preferably a polymeric layer former, on the osteoinductive and/or osteoconductive layer.

5. Medical implant according to either of Claims 3 and 4,
**characterized in that**
the antibiotic salts are gentamicin salts of palmitic acid, of lauric acid, of stearic acid, of oleic acid, of phenylbutyric acid, of naphthalene-1-carboxylic acid or sulfates of gentamicin.

6. Medical implant according to any of the preceding claims,
**characterized in that**
the active antibiotic ingredient which is slightly or poorly soluble in an aqueous environment is an antibiotic salt selected from the group of netilmicin myristate, sisomicin laurate, sisomicin myristate, sisomicin dodecyl sulfate, gentamicin laurate, gentamicin myristate, clindamycin laurate, amikacin laurate, amikacin myristate, amikacin dodecyl sulfate, kanamycin laurate, kanamycin myristate, kanamycin dodecyl sulfate, vancomycin laurate, vancomycin dodecyl sulfate, vancomycin myristate, vancomycin-teicoplanin, tobramycin laurate, tobramycin myristate, tobramycin dodecyl sulfate, ciprofloxacin laurate, ciprofloxacin myristate, clindamycin-teicoplanin, fusidic acid-gentamicin, fusidic acid-sisomicin, fusidic acid-netilmicin, fusidic acid-streptomycin, fusidic acid-tobramycin, fusidic acid-spectinomycin, fusidic acid-vancomycin, fusidic acid-ciprofloxacin, fusidic acid-moxifloxacin, fusidic acid-clindamycin, fusidic acid-lincomycin, fusidic acid-tetracycline, fusidic acid-chlortetracycline, fusidic acid-oxytetracycline and fusidic acid-rolitetracycline.

7. Medical implant according to any of the preceding claims,
**characterized in that**
the patchy overcoating of the osteoinductive and/or osteoconductive layer with the active antibiotic ingredient which is slightly or poorly soluble in an aqueous environment is achieved by in situ application with spaces being left free on the osteoinductive and/or osteoconductive layer, preferably by means of spraying, drop application or pipetting of a solution or suspension containing the active antibiotic ingredient.

8. Method for producing a coating on a medical implant, comprising the steps of:
- coating at least part of the surface of the medical implant with an osteoinductive and/or osteoconductive cover layer based on calcium phosphate and/or calcium carbonate,
- using an active antibiotic ingredient which is slightly or poorly soluble in an aqueous environment to overcoat the osteoinductive and/or osteoconductive layer in such a manner that patches are formed with spaces being left free on the osteoinductive and/or osteoconductive cover layer.

9. Method according to Claim 8,
**characterized in that**
the osteoinductive and/or osteoconductive cover layer based on calcium phosphate comprises hydroxylapatite.

10. Method according to either of Claims 8 and 9,
**characterized in that**
the active antibiotic ingredient comprises at least one antibiotic selected from the group consisting of:
- aminoglycoside antibiotics, preferably gentamicin and amikacin;
- lincosamide antibiotics, preferably clindamycin, lincomycin;
- cephalosporin antibiotics, preferably cefuroxime and cefoperazone;
- fluoroquinolone antibiotics, preferably ofloxacin,
- glycopeptide antibiotics, preferably vancomycin,
- □-lactam antibiotics, preferably ampicillin, or the salts thereof.

11. Method according to Claim 10,
**characterized in that**
the antibiotic or the salts thereof adhere(s) directly or via a support, preferably a polymeric layer former, on the osteoinductive and/or osteoconductive layer.

12. Method according to either of Claims 10 and 11,
**characterized in that**
the antibiotic salts are gentamicin salts of palmitic acid, of lauric acid, of stearic acid, of oleic acid, of phenylbutyric acid, of naphthalene-1-carboxylic acid or sulfates of gentamicin.

13. Method according to any of Claims 8-12,
**characterized in that**
the active antibiotic ingredient which is slightly or poorly soluble in an aqueous environment is an antibiotic salt selected from the group of netilmicin myristate, sisomicin laurate, sisomicin myristate, sisomicin dodecyl sulfate, gentamicin laurate, gentamicin myristate, clindamycin laurate, amikacin laurate, amikacin myristate, amikacin dodecyl sulfate, kanamycin laurate, kanamycin myristate, kanamycin dodecyl sulfate, vancomycin laurate, vancomycin dodecyl sulfate, vancomycin myristate, vancomycin-teicoplanin, tobramycin laurate, tobramycin myristate, tobramycin dodecyl sulfate, ciprofloxacin laurate, ciprofloxacin myristate, clindamycin-teicoplanin, fusidic acid-gentamicin, fusidic acid-sisomicin, fusidic acid-netilmicin, fusidic acid-streptomycin, fusidic acid-tobramycin, fusidic acid-spectinomycin, fusidic acid-vancomycin, fusidic acid-ciprofloxacin, fusidic acid-moxifloxacin, fusidic acid-clindamycin, fusidic acid-lincomycin, fusidic acid-tetracycline, fusidic acid-chlortetracycline, fusidic acid-oxytetracycline and fusidic acid-rolitetracycline.

14. Method according to any of Claims 8-13,
**characterized in that**
the patchy overcoating of the osteoinductive and/or osteoconductive layer with the active antibiotic ingredient which is slightly or poorly soluble in an aqueous environment is achieved by in situ application with spaces being left free on the osteoinductive and/or osteoconductive layer, preferably by means of spraying, drop application or pipetting of a solution or suspension containing the active antibiotic ingredient.

15. Medicinal implant obtainable by a method of Claims 8-14.

## Revendications

1. Implant médical, qui comprend au moins sur une partie de sa surface un revêtement ayant une couche de couverture ostéo-inductrice et/ou ostéo-conductrice, à base de phosphate de calcium et/ou de carbonate de calcium, **caractérisé en ce que** la couche de couverture ostéo-inductrice et/ou ostéo-conductrice est recouverte par zones, avec dégagement d'espaces intermédiaires sur la couche de couverture ostéo-inductrice et/ou ostéo-conductrice, d'un principe actif antibiotique, difficilement ou peu soluble en milieu aqueux.

2. Implant médical selon la revendication 1, **caractérisé en ce que** la couche de couverture ostéo-inductrice et/ou ostéo-conductrice à base de phosphate de calcium comprend de l'hydroxylapatite.

3. Implant médical selon l'une des revendications précédentes, **caractérisé en ce que** le principe actif antibiotique comprend au moins un antibiotique choisi dans le groupe consistant en :
- les antibiotiques aminoglycosidiques, de préférence la gentamicine et l'amikacine ;
- les antibiotiques lincosamides, de préférence la clindamycine, la lincomycine ;
- les antibiotiques céphalosporines, de préférence le céfuroxime et la céfopérazone ;
- les antibiotiques fluoroquinolones, de préférence l'ofloxazine,
- les antibiotiques glycopeptidiques, de préférence la vancomycine ;
- les antibiotiques □-lactame, de préférence l'ampicilline, ou les sels de ceux-ci.

4. Implant médical selon la revendication 3, **caractérisé en ce que** l'antibiotique ou les sels de celui-ci adhèrent directement, ou par l'intermédiaire d'un support, de préférence d'un agent filmogène polymère, à la couche ostéo-inductrice et/ou ostéo-conductrice.

5. Implant médical selon l'une des revendications 3 et 4, **caractérisé en ce que** les sels d'antibiotiques sont les sels de gentamicine de l'acide palmitique, de l'acide laurique, de l'acide stéarique, de l'acide oléique, de l'acide phénylbutyrique, de l'acide naphtalène-1-carboxylique ou les sulfates de gentamicine.

6. Implant médical selon l'une des revendications précédentes, **caractérisé en ce que** le principe actif antibiotique difficilement ou peu soluble en milieu aqueux est un sel d'antibiotique choisi dans le groupe consistant en le laurate de nétilmicine, le dodécylsulfate de nétilmicine, le myristate de nétilmicine, le laurate de sisomicine, le myristate de sisomicine, le dodécylsulfate de sisomicine, le laurate de gentamicine, le myristate de gentamicine, le laurate de clindamycine, le laurate d'amikacine, le myristate d'amikacine, le dodécylsulfate d'amikacine, le laurate de kanamycine, le myristate de kanamycine, le dodécylsulfate de kanamycine, le laurate de vancomycine, le dodécylsulfate de vancomycine, le myristate de vancomycine, la vancomycine-téicoplanine, le laurate de tobramycine, le myristate de tobramycine, le dodécylsulfate de tobramycine, le laurate de ciprofloxacine, le myristate de ciprofloxacine, la clindamycine-téicoplanine, l'acide fusidique-gentamycine, l'acide fusidique-sisomycine, l'acide fusidine-nétilmicine, l'acide fusidique-streptomycine, l'acide fusidique-tobramycine, l'acide fusidique-spectinomycine, l'acide fusidique-vancomycine, l'acide fusidique-ciprofloxacine, l'acide fusidique-moxifloxacine, l'acide fusidique-clindamycine, l'acide fusidique-lincomycine, l'acide fusidique-tétracycline, l'acide fusidique-chlortétracycline, l'acide fusidique-oxytétracycline et l'acide fusidique-rolitétracycline.

7. Implant médical selon l'une des revendications précédentes, **caractérisé en ce que** le revêtement par zones de la couche ostéo-inductrice et/ou ostéo-conductrice, comprenant le principe actif antibiotique difficilement ou peu soluble en milieu aqueux, est réalisé par application locale, avec dégagement d'espaces intermédiaires, sur la couche ostéo-inductrice et/ou ostéo-conductrice, de préférence par pulvérisation, en gouttes ou à la pipette, d'une solution ou d'une suspension contenant le principe actif antibiotique.

8. Procédé de fabrication d'un revêtement sur un implant médical, comprenant les étapes:
- revêtement d'au moins une partie de la surface de l'implant médical avec une couche de couverture ostéo-inductrice et/ou ostéo-conductrice à base de phosphate de calcium et/ou de carbonate de calcium,
- recouvrement de la couche ostéo-inductrice et/ou ostéo-conductrice avec un principe actif antibiotique difficilement ou peu soluble en milieu aqueux, de telle sorte qu'il se forme des zones par dégagement d'espaces intermédiaires sur la couche de couverture ostéo-inductrice et/ou ostéo-conductrice.

9. Procédé selon la revendication 8, **caractérisé en ce que** la couche de couverture ostéo-inductrice et/ou ostéo-conductrice à base de phosphate de calcium comprend de l'hydroxylapatite.

10. Procédé selon l'une des revendications 8 et 9, **caractérisé en ce que** le principe actif antibiotique comprend au moins un antibiotique choisi dans le groupe consistant en :
- les antibiotiques aminoglycosidiques, de préférence la gentamicine et l'amikacine ;
- les antibiotiques lincosamides, de préférence la clindamycine, la lincomycine ;
- les antibiotiques céphalosporines, de préférence le céfuroxime et la céfopérazone ;
- les antibiotiques fluoroquinolones, de préférence l'ofloxazine,
- les antibiotiques glycopeptidiques, de préférence la vancomycine ;
- les antibiotiques 0-lactame, de préférence l'ampicilline, ou les sels de ceux-ci.

11. Procédé selon la revendication 10, **caractérisé en ce que** l'antibiotique ou les sels de celui-ci adhèrent directement, ou par l'intermédiaire d'un support, de préférence d'un agent filmogène polymère, à la couche ostéo-inductrice et/ou ostéo-conductrice.

12. Procédé selon l'une des revendications 10 et 11, **caractérisé en ce que** les sels d'antibiotiques sont les sels de gentamicine de l'acide palmitique, de l'acide laurique, de l'acide stéarique, de l'acide oléique, de l'acide phénylbutyrique, de l'acide naphtalène-1-carboxylique ou les sulfates de gentamicine.

13. Procédé selon l'une des revendications 8 à 12, **caractérisé en ce que** le principe actif antibiotique difficilement ou peu soluble en milieu aqueux est un sel d'antibiotique choisi dans le groupe consistant en le laurate de nétilmicine, le dodécylsulfate de nétilmicine, le myristate de nétilmicine, le laurate de sisomicine, les myristate de sisomicine, le dodécylsulfate de sisomicine, le laurate de gentamicine, le myristate de gentamicine, le laurate de clindamycine, le laurate d'amikacine, le myristate d'amikacine, le dodécylsulfate d'amikacine, le laurate de kanamycine, le myristate de kanamycine, le dodécylsulfate de kanamycine, le laurate de vancomycine, le dodécylsulfate de vancomycine, le myristate de vancomycine, la vancomycine-téicoplanine, le laurate de tobramycine, le myristate de tobramycine, le dodécylsulfate de tobramycine, le laurate de ciprofloxacine, le myristate de ciprofloxacine, la clindamycine-téicoplanine, l'acide fusidique-gentamycine, l'acide fusidique-sisomycine, l'acide fusidine-nétilmicine, l'acide fusidique-streptomycine, l'acide fusidique-tobramycine, l'acide fusidique-spectinomycine, l'acide fusidique-vancomycine, l'acide fusidique-ciprofloxacine, l'acide fusidique-moxifloxacine, l'acide fusidique-clindamycine, l'acide fusidique-lincomycine, l'acide fusidique-tétracycline, l'acide fusidique-chlortétracycline, l'acide fusidique-oxytétracycline et l'acide fusidique-rolitétracycline.

14. Procédé selon l'une des revendications 8 à 13, **caractérisé en ce que** le revêtement par zones de la couche ostéo-inductrice et/ou ostéo-conductrice, comprenant le principe actif antibiotique difficilement ou peu soluble en milieu aqueux, est réalisé par application locale, avec dégagement d'espaces intermédiaires, sur la couche ostéo-inductrice et/ou ostéo-conductrice, de préférence par pulvérisation, en gouttes ou à la pipette, d'une solution ou d'une suspension contenant le principe actif antibiotique.

15. Implant médical pouvant être obtenu par un procédé selon les revendications 8 à 14.
